(19) **Europäisches Patentamt**

**European Patent Office**

**Office européen des brevets**

(11) **EP 1 054 895 B1**

(12) **EUROPEAN PATENT SPECIFICATION**

(45) Date of publication and mention
of the grant of the patent:
**22.11.2006 Bulletin 2006/47**

(51) Int Cl.:
*C07K 14/29* (2006.01)          *A61K 39/02* (2006.01)
*G01N 33/569* (2006.01)

(21) Application number: **99934287.6**

(22) Date of filing: **19.02.1999**

(86) International application number:
**PCT/SE1999/000230**

(87) International publication number:
**WO 1999/042479 (26.08.1999 Gazette 1999/34)**

(54) **NOVEL PEPTIDE DIAGNOSTIC REAGENT AND KIT FOR DETECTION OF RICKETTSIOSIS**

NEUES DIAGNOSTISCHES REAGENS UND KIT ZUR ERKENNUNG VON RICKETTSIOSE

REACTIF DE DIAGNOSTIC, PEPTIDE ET TROUSSE DE DETECTION DE LA RICKETTSIOSE

(84) Designated Contracting States:
**AT BE CH CY DE DK ES FI FR GB GR IE IT LI LU MC NL PT SE**

(30) Priority: **20.02.1998 SE 9800504**

(43) Date of publication of application:
**29.11.2000 Bulletin 2000/48**

(73) Proprietors:
• **Nilsson, Kenneth**
  **791 33 Falun (SE)**
• **Pahlson, Carl**
  **755 94 Uppsala (SE)**

(72) Inventors:
• **Nilsson, Kenneth**
  **791 33 Falun (SE)**
• **Pahlson, Carl**
  **755 94 Uppsala (SE)**

(74) Representative: **Aldenbäck, Ulla Christina**
**Dr. Ludwig Brann Patentbyra AB,**
**P.O. Box 1344**
**751 43 Uppsala (SE)**

(56) References cited:
• **JOURNAL OF BACTERIOLOGY, Volume 169, No. 6, June 1987, BURT E. ANDERSON et al., "Sequence Analysis of the 17-Kilodalton-Antigen Gene from Rickettsia Rickettsii", pages 2385-2390.**
• **EMBL, DATABASE GENBANK/DDBJ, Accession No. AF027124, 01-11-1997, BILLINGS A.N. et al., "Detection of a Spotted Fever Group Rickettsia in Amblyomma Cajennense (Acari: Ixodidae) in South Texas".**
• **JOURNAL OF BACTERIOLOGY, Volume 171, No. 9, Sept. 1989, BURT E. ANDERSON et al., "Comparative Sequence Analysis of a Genus-Common Rickettsial Antigen Gene", pages 5199-5201.**
• **JOURNAL OF CLINICAL MICROBIOLOGY, Volume 35, No. 1, January 1997, KENNETH NILSSON et al., "Characterization of a Spotted Fever Group Rickettsia from Ixodes Ricinus Ticks in Sweden", pages 243-247.**

**Description**

**Background of the invention**

The present invention relates to a novel peptide, diagnostic reagent and kit for detection of rickettsiosis, more closely for detection of tick borne *Rickettsia helvetica.* Furthermore, the invention relates to a vaccine against rickettsiosis.

Phylogenetically, life on earth can be devided into three major groups or kingdoms; the eucaryots, the eubacteria and the archea. Plants and higher organisms are the eucaryotes while the archea consist of bacteria adapted to extreme environments like hot springs, extreme saline's or the necessity to be able to use methane as carbon source in its catabolic processes. The vast majority of bacteria and the so called blue-green algae are the eubacteria. Of these a few (i.e. *Rickettsiae* and *Chlamydiae*) are so specialised that they are obligate intracellular parasites. The closest relatives to the *Rickettsiae* have been found in genetic evolutionary studies, and have been shown to in a far prehistoric time to be developed to become parasites or symbionts that live inside an other cell as an organelle like the mitochondria.

The genus *Rickettsiae* have until recently been considered to consists of three groups of strictly intracellular bacteria, namely the typhus (TG), the spotted fever (SFG) and the scrub typhus (STG) groups. The classification into species is based on the geographical distribution, arthropod hosts, intracellular location, structure of envelopes, conditions for cultivation and multiplication in chicken embryos, and serological patterns.

The TG includes the species *R. prowazekii* and *R. typhi.* The members of TG are found mainly in a cytoplasmatic location. Another common trait is the serological cross-reactivity in the Weil-Felix agglutination of *Proteus* OX19. The TG *Rickettsiae* occur in scattered locations worldwide and are transmitted to man by lice or fleas. The SFG *Rickettsiae* are mainly transmitted by ticks and about 20 different species of SFG have been described so far.

The species definition of SFG has been based on their serotype as determined by the complement fixation test, the toxin neutralization test in mice, the cross-immunity test in guinea pigs, or the microimmunofluorescence test (MIF). However, the serological differentiation of newly isolated strains are difficult, because of a significant cross-reactivity among the recognized SFG *Rickettsiae.*

The third group of *Rickettsiae,* the scrub typhus group, consists of only one described species, *Orientia tsutsugamushi.* This species occurs naturally in southern and south-eastern Asia and is transmitted by mite larvae (*Leptotrombidium spp.*). Furthermore, differences in the structure of the cell envelope and the ribosome sequences to those of the other groups have been recorded.

European ticks have been shown to harbor spotted fever *Rickettsiae* such as *R. helvetica* in Switzerland, *R. slovaca* in Russia and *R. conorii* in France.

The fact, that the clinical significance of rickettsial infections cannot be overestimated, has recently been emphasized. Many studies have shown that the signs and the symptoms of rickettsial diseases may differ widely.

This is valid for e.g. Rocky Mountain "Spotless" Fever, where the clinical findings are very similar to that of Lyme disease, but where the serological evaluation suggests an infection with *R. rickettsii.* Another example, is the study of a new rickettsial agent, "ELB", where the definitive clinical diagnosis was murine typhus. Extended investigations have now shown that the "ELB-agent" is a distinct rickettsial species with the proposed name *R. felis.*

Treatments of such infection are today almost exclusive effected by antibiotics or chemoteraperutic drugs. The most common problem with antibiotic treatment is ecological disturbances of the normal flora. A second, often more difficult, problem in treatment of bacterial infections caused by intracellular slow growing bacteria is that of obtaining high enough concentration of the drug at long time enough to kill bacteria in stationary phase of growth.

Present therapies for rickettsial infections are antibiotic treatments with chloramphenicol, tetracycline's and occasionally rifampicillin. Diagnosis of rickettsial infection can be made either by cultivation of the organisms or by diagnosis of body fluid from infected individuals, the latter being preferred because cultivation of *Rickettsiae* is often very difficult and hazardous.

For the extreme pathogenic *Rickettsiae,* not found in Europe, such as *R. prowazekii* (lice borne spot typhus), *R. rickettsii* (Rocky mountain spotted fever) and *Orientia tsutsugamushi* (Scrub tyfus) diagnostic reagents have been described. See, for example, US 7429936 which describes a recombinant protein for diagnosis of *R. rickettsii.* This protein can also be used to vaccinate humans against Rocky mountain spotted fever.

In *Journal of Bacteriology, Sept 1989, p 5199-5201,* the genes encoding the 17-kilodalton genus-common antigen have been cloned and sequenced from *R conorii, R prowazekii,* and *R typhi.* High degree of homology within the coding and control regions between the Rickettsial 17K genus-common antigens is presented in Fig.4.

In *Journal of Clinical Microbiology, Jan 1997, p 243-247,* the present inventors have shown that 1.7% of Scandinavian *Ixodes ricinus* ticks were positive for rickettsial DNA. Ticks are known to be the most common arthropod reservoirs and vectors of SFG *Rickettsiae.* Hitherto, *Rickettsia* are not known to be endemic or epidemic among humans or animals in Scandinavia.

The 16S rRNA gene from the isolated strain was amplified and sequenced, and a 1,380 bp sequence from this gene was compared with those of previously described rickettsial species. The result was a 100 % homology with *R.*

*helvetica.*

Species of the genus *Rickettsia* are closely related and the genetic differences between their 16S rRNA genes are only 2%. For example, 11 nucleotides differentiate this isolate from *R. conorii,* a species endemic in southern Europe and phylogenetically, the most closely related characterized spotted fever *Rickettsia,* and furthermore the only *Rickettsia* that has been found in *I. ricinus* so far. In comparison with *R. prowazekii,* which is less related and belongs to the typhus group, this strain differs in 25 nucleotide positions.

The RFLP pattern of the citrate synthetase gene improves the discrimination between Rickettsiales. The RFLP pattern obtained for the new strain, compared to those of known type strains, is most similar to that of SFG but distinctly different from the published pattern of *R. helvetica.* According to the nucleotide sequencing results, the detected *Rickettsia sp.* should be classified as a spotted fever (SFG) *Rickettsia.* Furthermore, the RFLP pattern suggests that the strain should be regarded as a subtype of *R. helvetica.*

## Summary of the invention

The present inventors have indications that the *Rickettsia* found in *I. ricinus* ticks are pathogenic for humans. Therefore, there exists a need of diagnosis and treatment of rickettsiosis in Scandinavia.

An object of the invention was to enable accurate diagnosis of rickettsial infection in Scandinavia. Another object was to provide effective treatment of infected individuals. These and other objects are accomplished according to the invention.

Thus, in a first aspect the invention relates to a peptide comprising the amino acid sequence as shown in Sequence Listing No. 1 wherein two ala-ala amino acids are present in the beginning of the peptide. The peptide is purified from natural sources, or produced by synthetic or recombinant means.

In a second aspect, the invention relates to a diagnostic reagent comprising the above peptide for detecting rickettsiosis, especially for detection of infection with *Rickettsia helvetica.*

In a third aspect, the invention relates to a diagnostic kit comprising the above diagnostic reagent which is in the form of a solution or bound to a solid phase. In one embodiment of this aspect, the diagnostic kit comprises a labelled anti-Ig antibody for detection of mammalian IgA, IgG or IgM antibodies against an antigen (ie. the above diagnostic reagent) from *R. helvetica.* The kit is preferably designed for conventional assay formats like ELISA, EIA, RIA, IRMA etc. The mammalian antibodies used as samples are collected from body fluids such as serum; blood, cerebrospinal fluid, tear fluid, seamen or sweat.

In a fourth aspect, the invention provides a vaccine comprising the above peptide for treatment of rickettsiosis. The treatment comprises administring, to an individual in need thereof, the vaccine in a suitable adjuvant in a pharmacologically effective amount to develop an immune response in said individual which protects said individual against rickettsiosis.

## Detailed description of the invention

The typical clinical picture during rickettsiosis is high fever, headache and rash. Depending on which rickettsial species that causes the disease the clinical feature could also include lymfadenopathia, myalgia, arthralgia and some forms present with neurological, renal and cardiac problems. The symptoms of the disease can vary from mild to severe and the duration will usually last for 2-3 weeks. The rareness of the disease, and the variety of symptomes complicate the diagnosis. Similar clinical pictures can appear during the course of some non-rickettsial bacterial and viral infections like meningococcemia, measles, typhoid fever, bacterial meningitis, secondary syfilis, leptospirosis, relapsing fever, infectious mononucleosis and rubella. If no rash occurs at all, all kinds of febrile illnesses have to be concerned.

Rickettsioses have also the inclination to become a latent infection, a hypothesis proven for *R prowazekii,* a discovery for which Henri Nicolle was awarded the 1928 year Nobel Prize in Physiology and Medicine. Such smouldering infection could after some years lead to severe sequelae. Isolates of *Rickettsiae* of unknown pathogenicity have a tendency to be considered non-pathogenic for human. During the last years a number of such "non-pathogenic" *Rickettsiae* have been proven to cause different diseases and is therefore reevaluated as pathogenic. Our hypothesis is that if the primary infection is mild, and therefore rarely or not at all become diagnosed, and still progresses into a latent infection causing serious sequelae these infections and organisms will become one of the most urgent to study.

Serological assays are the simplest diagnostic tests to perform, since serum can readily be sent to a reference laboratory. The more specific immunogenic reactions one can perform the the more specific will the test become and less crossreactions occur. The immunogenic quality of the bacteria are mainly associated with the structures exposed on the surface of the organism. Of such structures two groups are available. 1:One is the outer-membrane proteins, OMPs, and 2: The other is the LPS (lipo-poly-saccaride) as the *Rickettsiae* are Gram-negative bacteria. The LPS approach have been used for almost a century in the Weil-Felix reaction but is very unspecific. According to the invention, the OMP 17 kDa protein was used. The gene coding for this protein was amplified with the two specific primers in a standard PCR assay. The amplified PCR product was sequenced by using the Sanger dideoxy chain termination method and the result are given in Sequence Listing No. 2. This sequence was translated into amino acids, and a specific peptide was chosen corresponding to 28 amino acids from baspairs 277-360. This 28 amino acids antigenic peptide could be produced either by purification from cultured species closely related to *Rickettsia helvetica* expressing this peptide in the 17 kDa OMP, from recombinant organisms with a vector having an inserted sequence comprising bp 277-360 according to Sequence Listing No. 2 or variants thereof, or by pure chemical synthesis.

The invention will now be described more closely below in association with some non-limiting Examples along with the enclosed Sequence Listings and Figure.

**Example 1: Amplification and sequencing of rickettsial gene**

The following specific primer pair was constructed;

|  |  |
|---|---|
| 17up | 5'AAAATTCTAAAAACCAT |
| 17LOW | 5'TCAATTCACAACTTGCC |

From a collection of free living *I. ricinus* ticks, total DNA was isolated after trituration and addition of 300 $\mu$L low salt TE buffer (10 mM Tris pH 7.4, 1 mM EDTA, 10 mM NaCl), 20 $\mu$L 20% sodium dodecyl sulphate (SDS) and 5 $\mu$L 10 mg/ml Proteinase K (Boehringer, Mannheim). The mixture was incubated at 55°C for 1 h and heated for 10 min at 95°C. Extraction was performed twice with saturated phenol, once with a mixture of phenol/chloroform and finally once with chloroform. The DNA was precipitated overnight by addition of 1/10 vol. 4 M sodium acetate and 3 vol. 99% ethanol and collected by centrifugation at 20,000 rpm in a microcentrifuge. The pellet was washed with 70% alcohol and then dried under vacuum and dissolved in 50 $\mu$L distilled water and used as template for PCR.

**PCR amplification**. The primers described above were used with thermal cycle conditions 93°C 5 min.(93°C 30 sec.-52°C 60 sec.-72°C 30 sec) repeated 35 times, yielded an approximately 530 base pair (bp) fragment covering the complete gene, encoding for the 17 kDa outer membrane protein and flanking regions.

The general chemical conditions for the PCR amplification consisted of a mixture of 1U Taq DNA polymerase (Boehringer, Mannheim) 2.5 $\mu$L 10 times buffer supplied with the enzyme, 1$\mu$L 25mM MgCl$_2$, 2.5$\mu$L 2 mM dNTP's, 5 pmol of each primer, 1 $\mu$L DNA template and double distilled water to 25 $\mu$L. The reaction was performed in a Perkin Elmer 9600 thermocycler, and the amplified products were analyzed on a 1.5% agarose (Kodak) gel in 0.5 Tris Borate EDTA (TBE) buffer. Chosen as positive PCR control, was *Rickettsia prowazekii* purified DNA. As negative controls PCR buffer treated the same way as the ticks were included.

The major part of the coding sequence of this gene is shown in Sequence Listing No. 2 and was performed by direct solid phase DNA sequencing. Immobilization of the biotinylated PCR products followed by strand separation and template preparation, was performed with super paramagnetic beads, Dynabeads M-280 Streptavidin (Dynal, Oslo, Norway). The nucleotide sequence of the 17 kDa OMP gene was determined in both directions by automated solid phase DNA sequencing with the ALF (Automated Laser Fluorescence) system (Pharmacia Biotech, Uppsala, Sweden). Sequencing was also performed manually with $^{35}$S dATP (Amersham) and Sequenace II (USAB, Ohio) according to the instructions of the suppliers.

**Example 2: Construction of antigenic rickettsial peptide**

A theoretical calculation of the antigenic domains was performed using Jameson-Wolf algorithm in the DNA StarData-program. The Jameson-Wolf produces an index of antigenicity by combining values for hydrophilicity (Hoop-Woood H ( <2> 0,5 <1> 0 <-1> -0,5 <-2>)), Surface Probability (Emini S ( <1> 1.0 <0>)), Flexibility (Karplans -Shultz F (<1> 1.0 <0>)) and the secondary structure predictions of Chou-Fasman (CF (T=2, t=1, 0) and Garnier- Robson (GR (T=2,t=1, 0,3) by the following formula

$$A = 0,3H + 0,15S + 0,15F + 0,2CF + 0,2\ GR$$

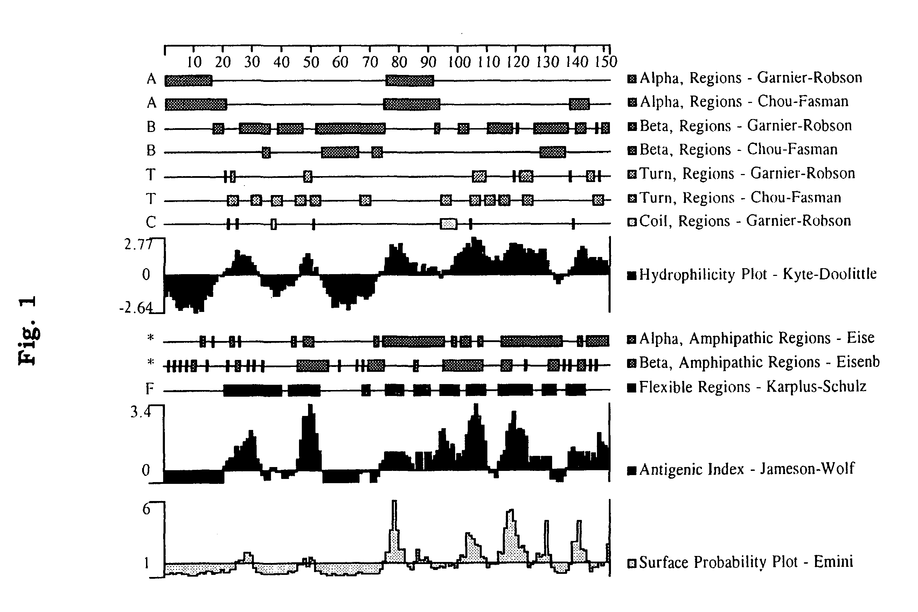

The method produces a graph, see Fig. 1, that indicates putative antigenic sites at peak valued residues. The peak broadening function adds a cascade of 20%, 40%, 60%, 80%, 100%, 80%, 60%, 40%, 20%, the peak value to the nine residue values surrounding each local maximum of the result.

In the antigenic index graph, the peptide sequence according to the invention corresponds to the region of amino acids 93-120, with three high peak residues expressing high antigenicity. This 28 amino acid peptide is disclosed in Sequence Listing No. 1.

A confirming method for visualising the antigenicity is the Western blot assay. 5 μL of purified organism was dissolved in a Laemmli solution (4%SDS,10% 2-mercaptoethanol, 0,5 % bromophenol blue, 0,125 M Tris hydrochloride (pH 6.8), 25% glycerol) at room temperature, and SDS-PAGE was carried out with a 7.5% separating gel and 3.9 stacking gel. The gel was run in a Mini-Protein II cell (Bio-Rad) at 10 mA in an ice-bath, and protein bands were visualized by Coomassie blue staining. A high-range molecular weight standard (Bio-Rad) was used to estimate the molecular weights of the electrophoretic bands. Another identical gel was transferred to nitrocellulose paper in a Trans-blot apparatus (Bio-Rad) at 50 V for 1 h in an ice bath. Non-specific binding sites were blocked overnight with 5% non-fat dry milk-TBS (10mM Tris hydrochloride (pH 7.5), 250 mM NaCl, 0,01% Merthiolate). After three 10-min washes in TBS, the nitrocellulose paper was overlaid with antisera from index patient, diluted 1/100 in 3% non-fat dry milk-TBS, and rocked for 2 h. Reactive antibodies were detected with 1/200 gamma chain specific anti-human Ig (Bio Merieux, Marcy lÉtoile, France) in 3% nonfat dry milk-TBS. After three further washes in TBS, the bound peroxidase was detected with a solution containing 0,015% 4-chloro-1-naphthol, 0,015% hydrogen peroxide, and 16% methanol in TBS. As soon as the bands became visible, the reactions were stopped with repeated washes in distilled water.

As a result, a distinct band appears at 17 kD.

### Example 3: Diagnostic use of the peptide of the invention

A diagnostic reagent comprising the antigenic part of said OMP can be used for detecting/diagnosing/quantifying antigenic response in humans and other mammals after contact with bacteria closely related to *R. helvetica* which express a protein comprising the peptide of the invention. The diagnostics can be performed by a variety of methods for example; Indirect immunofluorescense; Enzyme immunoassays; Radio immunoassays Immunodiffusion; Western blot assays ( see example 2).

The two Ala-Ala aminoacids in the beginning of the peptide of the invention make the peptide very suitable for coupling to a solid phase. These two hydrophobic aminoacids will reduce unspecific binding and, thereby, improve the sensitivity of the diagnostic test.

Serum from an infected patient gave a clear serological response with the diagonostic kit of the invention indicating that *R. helvetica* caused the infection. The result was verified with PCR and immunohistochemistry.

Serum from the same patient has also been shown to react with the peptide of the invention in immunodiffusion experiments.

As an alternative, the cellular response can be tested by, for example, an Immuno spot assay.

### Example 4: Therapeutic use of the peptide of the invention

The antigenic part of said OMP could also be used mixed or coupled to an adjuvant (for example Freunds or Iscomes) to be used as a vaccine. The vaccine can either be used therapeutically or prophylactically.

### SEQUENCE LISTINGS

Information for Sequence Listing No. 1:
Length: 28 aa
Type: amino acid

```
    Ala Ala Pro Ser Gly Ser Asn Val Glu Trp Arg Asn Pro Asp Asn
                      5                   10                      15

    Gly Asn Tyr Gly Tyr Val Thr Pro Asn Lys Thr Tyr Arg
                     20                   25
```

Information for Sequence Listing No. 2:
Length: 454 bp
Type: DNA

```
ATG ATA CTA TTA TCT AAA ATT ATG ATT ATA GCT CTT GCA GCT TCT
                                            30

ATG TTA CAA GCC TGT AAC GGT CCG GGT GGT ATG AAT AAA CAA GGT
            60                                              90

ACA GGA ACA CTT CTT GGC GGT GCC GGC GGT GCA TTA CTT GGT TCT
                                        120

CAA TTT GGT AAA GGT AAA GGG CAA CTT GTC GGA GTA GGT GTA GGT
            150                                            180

GCA TTA CTT GGA GCA GTT CTT GGC GGG CAA ATC GTT GCA GGT ATG
                                        210

GAT GAG CAG GAT AGA AGA CTT GCA GAG CTT ACC TCA CAG AGA GCT
            240                                            270

TTA GAA GCA GCT CCT AGC GGT AGT AAC GTA GAG TGG CGT AAT CCG
                                        300

GAT AAC GGC AAT TAC GGT TAC GTA ACA CCT AAT AAA ACT TAT AGA
            330                                            360

AAT AGC ACT GGT CAA TAT TGC CGT GAG TAC ACT CAA ACA GTT GTA
                                        390

ATA GGC GGA AAA CAA CAA AAA GCA TAC GGT AAT GCA TGC CGC CAA
            420                                            450

CCT G
```

## Claims

1. A peptide containing the amino acid sequence shown in Sequence Listing No. 1 wherein two ala-ala amino acids are present in the beginning of the peptide.

2. A peptide according to claim 1, which is purified from natural sources, or produced by synthetic or recombinant means.

3. A gene encoding the peptide according to claim 1 or 2 comprising all variants due to the degeneracy of the genetic code.

4. A diagnostic reagent comprising the peptide according to claim 1 or 2 for detecting rickettsiosis.

5. A diagnostic reagent according to claim 4, for detection of infection with *Rickettsia helvetica* expressing the peptide according to claim 1.

6. A diagnostic kit comprising a diagnostic reagent according to any one of claims 4-5.

7. A diagnostic kit according to claim 6, wherein the reagent is in the form of a solution or bound to a solid phase.

8. A diagnostic kit according to any of claims 6-7 , comprising a labelled anti-Ig antibody for detection of mammalian IgA, IgG or IgM antibodies against an antigen from *R. helvetica.*

9. A diagnostic kit according to claims 6-7, for detection of cellular immune response against an antigen from *R. helvetica.*

10. A vaccine comprising the peptide according to claim 1 or 2 optionally mixed or coupled to an adjuvant for prevention of rickettsiosis.


**Patentansprüche**

1. Peptid umfassend die Aminosäuresequenz des Sequenzprotokolls No. 1, wobei zwei Ala-Ala Aminosäuren am Anfang des Peptids vorhanden sind.

2. Das Peptid gemäß Anspruch 1, das aus natürlichen Quellen gereinigt oder mittels synthetischer oder recombinanter Methoden hergestellt wird.

3. Gen, das für das Peptid gemäß Anspruch 1 oder 2 kodiert, umfassend alle Varianten infolge der Degeneriertheit des genetischen Codes.

4. Diagnostisches Reagens umfassend das Peptid gemäß Anspruch 1 oder 2 zur Detektion von Rickettsiose.

5. Das diagnostische Reagens gemäß Anspruch 4 zur Detektion einer Infektion mit das Peptid gemäß Anspruch 1 exprimierender *Rickettsia helvetica.*

6. Diagnostischer Kit umfassend ein diagnostisches Reagens gemäß einem der Ansprüche 4 oder 5.

7. Der diagnostische Kit gemäß Anspruch 6, wobei das Reagens eine Lösung ist oder an eine feste Phase gebunden ist.

8. Der diagnostische Kit gemäß einem der Ansprüche 6 oder 7, umfassend einen markierten Anti-Ig-Antikörper zur Detektion humaner IgA, IgG oder IgM Antikörper gegen ein Antigen von *R. helvetica.*

9. Der diagnostische Kit gemäß einem der Ansprüche 6 oder 7 zur Detektion einer zellulären Immunantwort gegen ein Antigen von *R. helvetica.*

10. Impfstoff zur Prävention von Rickettsiose, wobei der Impfstoff das Peptid gemäß Anspruch 1 oder 2 umfasst, das mit einem Adjuvans gemischt oder an ein Adjuvans gekoppelt sein kann.


**Revendications**

1. Peptide contenant la séquence d'acides aminés représentée sur la liste de séquences N° 1 où deux acides aminés ala-ala sont présents au commencement du peptide.

2. Peptide selon la revendication 1, qui est purifié à partir de sources naturelles, ou produit par un moyen de synthèse ou un moyen recombinant.

3. Gène codant pour le peptide selon la revendication 1 ou 2, comprenant toutes les variantes dues à la dégénérescence du code génétique.

4. Réactif de diagnostic comprenant le peptide conforme à la revendication 1 ou 2 destiné à détecter la rickettsiose.

5. Réactif de diagnostic selon la revendication 4, destiné à détecter une infection avec *Rickettsia helvetica* exprimant le peptide conforme à la revendication 1.

6. Nécessaire de diagnostic comprenant un réactif de diagnostic selon l'une quelconque des revendications 4 à 5.

7. Nécessaire de diagnostic selon la revendication 6, dans lequel le réactif se présente sous la forme d'une solution ou bien est lié à une phase solide.

**8.** Nécessaire de diagnostic selon l'une quelconque des revendications 6 à 7, comprenant un anticorps anti-Ig marqué pour la détection d'anticorps IgA, IgG ou IgM de mammifères contre un antigène provenant de *Rickettsia helvetica.*

**9.** Nécessaire de diagnostic selon les revendications 6 à 7, destiné à la détection d'une réponse immunitaire cellulaire contre un antigène provenant de *Rickettsia helvetica.*

**10.** Vaccin comprenant le peptide selon la revendication 1 ou 2 optionnellement mélangé ou couplé à un adjuvant destiné à la prévention de la rickettsiose.

# Fig. 1

Alpha, Regions - Garnier-Robson
Alpha, Regions - Chou-Fasman
Beta, Regions - Garnier-Robson
Beta, Regions - Chou-Fasman
Turn, Regions - Garnier-Robson
Turn, Regions - Chou-Fasman
Coil, Regions - Garnier-Robson

Hydrophilicity Plot - Kyte-Doolittle

Alpha, Amphipathic Regions - Eise
Beta, Amphipathic Regions - Eisenb
Flexible Regions - Karplus-Schulz

Antigenic Index - Jameson-Wolf

Surface Probability Plot - Emini